# EUROPEAN PATENT APPLICATION

(11) **EP 1 618 909 A1**
(43) Date of publication of application: **25.01.2006**
(21) Application number: 03723115.6
(22) Date of filing: 15.04.2003
(51) Int. Cl.: A61M 5/34

(54) **MEDICAL ANESTHETIC NEEDLE**

(71) Applicant: Doctor Japan Co., Ltd., Shinjuku-ku, Toyko 160-0022 (JP)
(72) Inventor: HIGUCHI, Akio, Doctor Japan Co., Ltd., Shinjuku-ku, Tokyo 160-0022 (JP); HYUGAJI, Hayato, Doctor Japan Co., Ltd., Shinjuku-ku, Tokyo 160-0022 (JP)
(74) Representative: Altenburg, Udo
(86) International application number: PCT/JP2003/004757
(87) International publication number: WO 2004/091702

(57) **Abstract**

An anesthetic needle for medical use that is extremely easy to use is provided. The anesthetic needle includes an epidural needle (1) and an anesthetic needle (40) that is rotatably inserted into the epidural needle from a proximal end portion. The epidural needle is provided, at the proximal end portion, with a length adjusting mechanism (10) of a tube shape extending in an axial direction. The anesthetic needle is provided, at a proximal end portion, with an engaging portion (44) of a circular tube shape loosely fitted within the length adjusting mechanism of the epidural needle. A needle is inserted through a center portion of the engaging portion. A plurality of circumferential grooves (45) are juxtaposed axially on an outer peripheral surface of the engaging portion. The length adjusting mechanism is provided therein with an engaging projection (17). The engaging projection engages with the circumferential grooves to inhibit the axial movement of the anesthetic needle, while being slidable circumferentially with the circumferential grooves.

## Description

### Technical Field

The present invention relates to an anesthetic needle for medical use including an epidural needle and an anesthetic needle.

### Background Art

Two conventional methods of injecting an anesthetic in a spinal space or the like of a patient are known. One uses an epidural needle to deliver medicament to an epidural space of the patient. The other uses a needle of a relatively small diameter to directly deliver the medicament into a subarachnoid space of a spinal column or the like of the patient. In the former method, however, the medicament must percolate through semi-liquid fat to reach nerve roots. This can at times retard the onset of an anesthetic block. Moreover a potential exists for toxicity caused by large doses of medicament necessary to obtain a sufficient block. As regards the latter method, on the other hand, a dura mater must be punctured in order to allow the needle to reach the subarachnoid space. Then, a cerebrospinal fluid leaks through this punctured hole, causing the patient to develop a postoperative headache. In addition, it defies the use of a catheter for extending the anesthetic block.

A CSE method was developed to solve these problems. A procedure according to the CSE method is carried out as detailed in the following. Specifically, an epidural needle is inserted in the patient and advanced onto the epidural space without puncturing the dura mater. An anesthetic needle having a small diameter is inserted into the epidural needle. The anesthetic needle is further inserted until a distal end thereof punctures the dura mater and reaches the subarachnoid space. When it is felt that the distal end of the anesthetic needle has been inserted into the subarachnoid space, a stylet of the anesthetic needle is removed and it is confirmed if there is a flashback from a proximal end of the anesthetic needle. The anesthetic is delivered. The anesthetic needle is pulled out as necessary and an epidural catheter is inserted in the epidural space through the epidural needle.

As described in the foregoing, the procedure according to the CSE method uses two needles having different diameters from each other that are inserted and moved relative to each other. Since the anesthetic needle is slidable within the epidural needle, however, the anesthetic needle can be displaced during administration of the anesthetic. In addition, whether the anesthetic needle has been sufficiently inserted or not through the dura mater can be determined by means of the flashback. Meanwhile, the thickness of the dura mater, and the distance to the subarachnoid space, will vary from one human body to another. It therefore becomes necessary to check for flashback by, for example, turning the anesthetic needle in units of 90°.

In addition, in the CSE method, fitting portions of the epidural needle and the anesthetic needle are formed to comply with an international standard (ISO: 592-2) to ensure that any epidural needle or anesthetic needle manufactured and sold by one manufacturer can be used with any other needle manufactured and sold by a different manufacturer. The anesthetic needle for medical use described in Japanese Patent No. 2787012 has been devised to solve the aforementioned problems. A locking mechanism is interposed between the epidural needle and the anesthetic needle formed in compliance with the international standard. The locking mechanism inhibits an axial movement of the anesthetic needle relative to the epidural needle after the position of the anesthetic needle has been properly adjusted. Once the locking mechanism has been actuated, the anesthetic needle can be rotated integrally with the locking mechanism relative to the epidural needle.

In accordance with the conventional anesthetic needle for medical use disclosed in the Japanese Patent No. 2787012, however, the anesthetic needle can only be rotated integrally with the locking mechanism relative to the epidural needle. The locking mechanism has, because of its construction involved, a substantial size and a substantial mass as compared with the anesthetic needle. This prevents a practitioner from turning the anesthetic needle with an exquisite tactile sense. Should he or she be able to turn the anesthetic needle, the rotation can never be smooth. Another problem is that it is not easy for the practitioner to gain a sense that the anesthetic needle has punctured into the subarachnoid space because of a sliding pressure involved with the anesthetic needle that slides within the locking mechanism.

Referring to the conventional anesthetic needle for medical use shown in FIG. 14, a small-diameter portion 103b of a locking mechanism 103 provided for inhibiting axial movement of an anesthetic needle 102 relative to an epidural needle 101 is integrally mounted on the epidural needle 101 for the exclusive use for this conventional anesthetic needle for medical use. The anesthetic needle 102 is rotatably mounted on a proximal end portion of a large-diameter portion 103a of the locking mechanism 103. The large-diameter portion 103a is screwed onto the small-diameter portion 103b of the locking mechanism 103 after the position of the anesthetic needle 102 has been adjusted. This inhibits the axial movement of the anesthetic needle relative to the epidural needle. Even after this locking operation, the anesthetic needle 102 can still be rotated relative to the epidural needle.

With the conventional anesthetic needle for medical use shown in FIG. 14, however, the large-diameter portion 103a of the locking mechanism 103 slides over an outside of the small-diameter portion 103b. The same problem as in the aforementioned anesthetic needle for medical use then surfaces that it is not easy for the practitioner to gain the sense that the anesthetic needle has punctured into the subarachnoid space. Moreover, the small-diameter portion 103b of the locking mechanism 103 is integrally formed with the dedicated epidural needle 101. This presents another problem that the locking mechanism cannot be mounted on an epidural needle manufactured in compliance with the international standard by a different manufacturer.

### Disclosure of the Invention

The present invention has been made to solve the aforementioned problems.

It is therefore an object of the present invention to provide an extremely-easy-to-use anesthetic needle for medical use that provides the practitioner with a positive tactile feedback of the anesthetic needle's puncture into the subarachnoid space and that allows the practitioner to smoothly rotate the anesthetic needle with an exquisite tactile sense, while inhibiting the axial movement of the anesthetic needle relative to the epidural needle after the position of the anesthetic needle has been adjusted. It is another object of the present invention to provide an anesthetic needle for medical use that can be used with an epidural needle manufactured by any given manufacturer in compliance with the required international standard.

To achieve the foregoing objects, there is provided in accordance with the present invention an anesthetic needle for medical use provided with an epidural needle and an anesthetic needle rotatably inserted into the epidural needle from a proximal end portion. The anesthetic needle for medical use is characterized in the following aspects. Specifically, the epidural needle is provided, at the proximal end portion, with a length adjusting mechanism of a tube shape extending in an axial direction. The anesthetic needle is provided, at a proximal end portion, with an engaging portion of a circular tube shape loosely fitted within the length adjusting mechanism. A needle is inserted through a center portion of the engaging portion. A plurality of circumferential grooves are juxtaposed axially on an outer peripheral surface of the engaging portion. The length adjusting mechanism is provided therein with an engaging projection. The engaging projection engages with the circumferential grooves to inhibit the axial movement of the anesthetic needle. Meanwhile, the engaging projection is slidable circumferentially with the circumferential grooves.

In the anesthetic needle for medical use in accordance with the present invention, the anesthetic needle is provided with the engaging portion loosely fitted within the length adjusting mechanism mounted on the epidural needle. This helps reduce a sliding pressure between the length adjusting mechanism and the engaging portion. This, in turn, allows the practitioner to gain a positive sense that the anesthetic needle has punctured into the subarachnoid space. In addition, the engaging projection is engaged with the circumferential grooves in the engaging portion after the position of the anesthetic needle has been adjusted. The axial movement of the anesthetic needle relative to the epidural needle can thereby be inhibited. In the meantime, the engaging projection is slidable circumferentially with the circumferential grooves. This allows the anesthetic needle to rotate relative to the epidural needle. Furthermore, the engaging portion can be formed into a thin, lightweight body, since there is only a needle disposed therein. The anesthetic needle can therefore be rotated smoothly with an exquisite tactile sense.

The epidural needle may be coupled to the length adjusting mechanism by fitting a convex portion formed at the proximal end portion of the epidural needle into a concave portion formed at a distal end portion of the length adjusting mechanism. If the concave portion of the length adjusting mechanism is manufactured in compliance with the required international standard, the anesthetic needle for medical use in accordance with the present invention can be mounted with an epidural needle manufactured by any given manufacturer having the convex portion manufactured in compliance with the aforementioned international standard.

The circumferential grooves may be formed into an annular form or external threads. If the circumferential grooves are formed annularly, flashback can be confirmed without allowing the anesthetic needle to move in the axial direction. This is achieved by engaging the engaging projection with the circumferential grooves in the engaging portion and allowing the anesthetic needle to rotate relative to the epidural needle. If the circumferential grooves are formed into external threads, on the other hand, the axial position of the anesthetic needle can be fine-adjusted for the following action. Specifically, when the anesthetic needle is rotated, it causes the engaging projection to slide along the circumferential grooves formed in external threads, thus moving the axial position of the anesthetic needle slightly relative to the epidural needle.

The length adjusting mechanism may, for example, be provided with a first engaging member that crosses the engaging portion and is inserted into the length adjusting mechanism movably between an engaging position and a disengaging position. The engaging projection is provided in a protruding manner on the engaging portion side of the first engaging member.

The first engaging member may be urged in the direction toward the engaging position by a spring. Given such an arrangement, the tension of the spring causes the engaging projection to be automatically engaged with the circumferential grooves during engagement and ensures a positive retention of engagement therebetween. This makes the anesthetic needle for medical use according to the present invention even easier to use.

The length adjusting mechanism may, for example, be provided with a second engaging member that is formed into external threads and screwed into the length adjusting mechanism so as to freely advance and retract radially between the engaging position and the disengaging position. The engaging projection is disposed at a distal end portion of the second engaging member.

The length adjusting mechanism may, for example, be provided with a third engaging member including a bar member that crosses the engaging portion and is inserted into the length adjusting mechanism movably between the engaging position and the disengaging position and a rotary plate that has screw threads thereon for engaging the bar member and turns for moving the bar member. The engaging projection is disposed on the engaging portion side of the bar member.

The length adjusting mechanism may, for example, be provided with a fourth engaging member that crosses the engaging portion and is journaled in the length adjusting mechanism oscillatably between the engaging position and the disengaging position. The engaging projection is disposed on the engaging portion side of the fourth engaging member.

The length adjusting mechanism may be provided with a window portion, through which the engaging portion can be viewed. Through this arrangement, it is possible to easily determine the position of the engaging portion and the like through the window portion.

The length adjusting mechanism may be formed from a transparent material. Through this arrangement, it is possible to determine even more easily the position of the engaging portion and the like.

The length adjusting mechanism may be provided with a scale, with which the axial movement of the anesthetic needle can be read. Through this arrangement, it is possible to determine numerically the position of the engaging portion and the like.

Through these arrangements, the anesthetic needle for medical use in accordance with the present invention allows the practitioner to gain a positive sense that the anesthetic needle has punctured into the subarachnoid space. The anesthetic needle for medical use in accordance with the present invention can also be rotated smoothly with an exquisite tactile sense, while inhibiting the axial movement of the anesthetic needle relative to the epidural needle after the position of the anesthetic needle has been adjusted. This produces an outstanding effect that the anesthetic needle for medical use in accordance with the present invention is extremely easy to use. It also yields another outstanding effect that the anesthetic needle for medical use in accordance with the present invention can be used with an epidural needle manufactured by any given manufacturer in compliance with the required international standard.

### Brief Description of the Drawings

FIG. 1 is a perspective view showing an anesthetic needle for medical use in accordance with the present invention;
FIG. 2 is a front cross-sectional view showing the anesthetic needle for medical use shown in FIG. 1;
FIG. 3 is a plan view showing a length adjusting mechanism;
FIG. 4 is a plan view showing another length adjusting mechanism;
FIG. 5 is a schematic view showing an engaged condition between circumferential grooves and an engaging projection;
FIG. 6 is a longitudinal cross-sectional view showing a first engaging member;
FIG. 7 is a schematic view showing an engaged condition between another circumferential grooves and another engaging projection;
FIG. 8 is a longitudinal cross-sectional view showing another first engaging member;
FIG. 9 is a longitudinal cross-sectional view showing a second engaging member;
FIG. 10 is a plan cross-sectional view showing a third engaging member;
FIG. 11 is a cross-sectional view taken along line XI-XI of FIG. 10 showing the third engaging member;
FIG. 12 is a plan view showing a fourth engaging member;
FIG. 13 is a cross-sectional view taken along line XIII-XIII of FIG. 12 showing the fourth engaging member; and
FIG. 14 is a front elevational view showing a conventional anesthetic needle for medical use.

### Best Mode for Carrying out the Invention

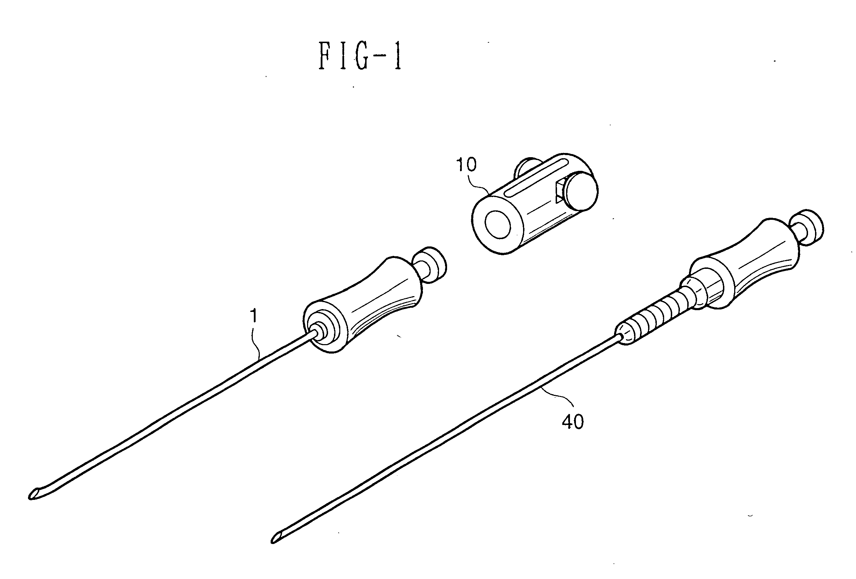

The anesthetic needle for medical use in accordance with the preferred embodiments of the present invention will be described in detail with reference to FIGS. 1 through 13.

Referring to FIG. 1, the anesthetic needle for medical use includes an epidural needle 1, and an anesthetic needle 40 inserted into the epidural needle 1 from the side of a proximal end portion. The epidural needle 1 is provided, at a proximal end portion thereof, with a length adjusting mechanism 10 of a tube form extending in an axial direction.

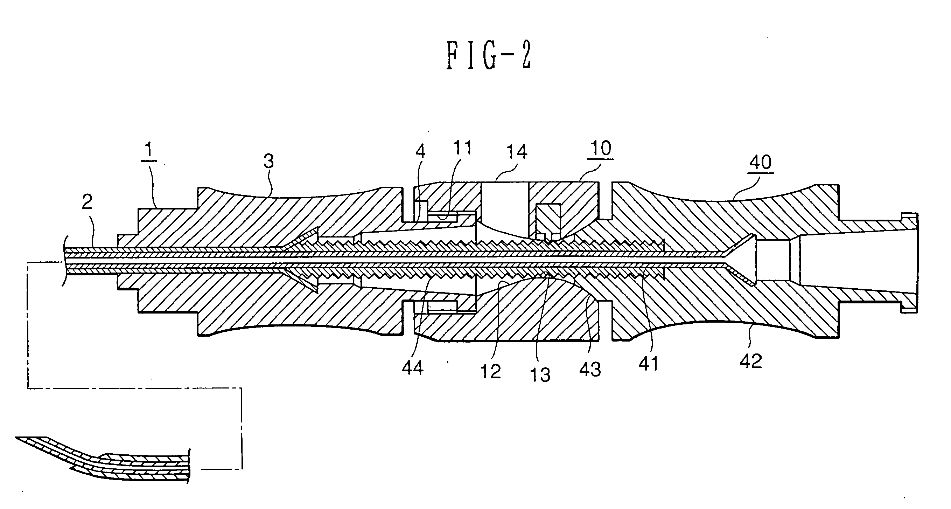

Referring to FIG. 2, the epidural needle 1 includes a needle 2 of a stainless-steel hollow straight tube and a tab 3. A convex fitting (convex portion) 4 in compliance with the international standard (ISO: 592-2) is coaxially disposed on a proximal end portion of the tab 3. A concave fitting (concave portion) 11 in compliance with the aforementioned international standard is coaxially disposed on a distal end portion of the length adjusting mechanism 10. An internal thread portion formed by a double-start thread is formed on an inside of the concave fitting 11. A flange portion of the convex fitting 4 of the epidural needle 1 is screwed into the internal thread portion of the concave fitting 11 of the length adjusting mechanism 10 so that the convex fitting 4 fits into the concave fitting 11. The epidural needle 1 is thereby coupled to the length adjusting mechanism 10.

As described in the foregoing, the length adjusting mechanism 10 can be mounted with the epidural needle 1 of any given manufacturer having the convex fitting 4 manufactured in compliance with the aforementioned international standard. The system of fit between the convex fitting and the concave fitting is not necessarily limited to the aforementioned screwing. Rather, any other known system of fit may be employed, instead. Further, the type of coupling between the epidural needle and the length adjusting mechanism is not limited to the aforementioned fit achieved between the convex fitting and the concave fitting, either.

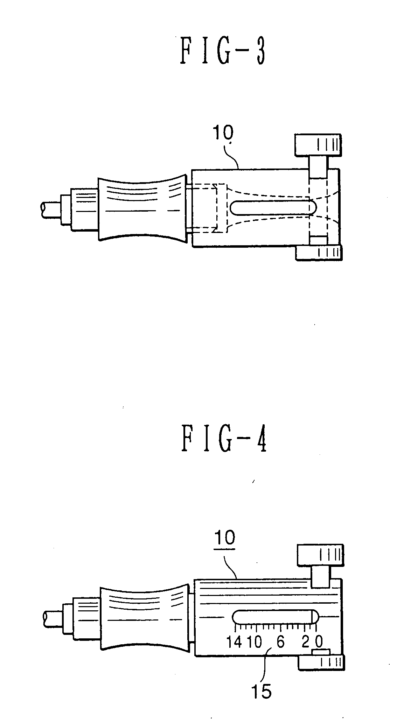

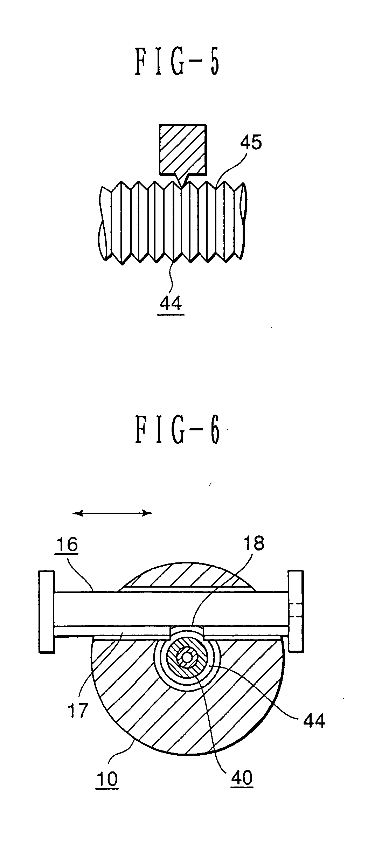

An insertion hole 12 having a convergent-divergent shape and having a throat portion 13 forming the minimum cross-sectional area is provided coaxially inside the length adjusting mechanism 10. A window portion 14 providing communication between an outer peripheral portion and the insertion hole 12 is also provided. Referring to FIG. 3, the length adjusting mechanism 10 is formed from a transparent material of a resin or the like. As shown in FIG. 4, a scale 15 providing a reading of the axial movement of the anesthetic needle may be provided near the window portion 14 of the length adjusting mechanism 10.

Referring to FIG. 2, an anesthetic needle includes a needle 41 of a stainless-steel hollow straight tube and a tab 42. A convex portion 43 to mate with an internal shape of the insertion hole 12 of the length adjusting mechanism 10 is formed on a distal end portion of the tab 42. An engaging portion 44 of a circular tube shape, through which the needle 41 is inserted, is disposed on a distal end of the convex portion 43. The engaging portion 44 is integrally implanted with the needle 41 in the distal end portion of the tab 42. The needle 41 passes through the tab 42.

Referring to FIG. 5, a plurality of annular circumferential grooves 45 are juxtaposed, axially equally spaced on an outer peripheral surface of the engaging portion 44. As shown in FIG. 2, the engaging portion 44 can be inserted integrally with the needle 41 into the insertion hole 12 of the length adjusting mechanism 10 from the proximal end portion. The gap between the engaging portion 44 and the insertion hole 12 is the minimum at the throat portion 13 of the insertion hole 12. There is nonetheless a slight gap even at the throat portion 13. As such, the anesthetic needle'40 is loosely fitted within the length adjusting mechanism 10. This allows the anesthetic needle 40 to be advanced, retracted, or rotated with a minimum of sliding pressure.

Referring to FIG. 6, the length adjusting mechanism 10 is provided with a bar member 16 that crosses the engaging portion 44 of the anesthetic needle 40 and passes therethrough so that the bar member 16 can be moved, but not be rotated. An engaging projection 17 is provided in a protruding manner on the side of the engaging portion 44 of the bar member 16. The engaging projection 17 extends in a longitudinal direction of the bar member 16 with one discontinued portion 18 therein. As shown in FIG. 5, the bar member 16 engages the circumferential grooves 45 of the engaging portion 44 of the anesthetic needle 40 by means of the engaging projection 17. The engaging projection 17 is disengaged as the bar member 16 moves to the discontinued portion 18.

The operating procedures of the anesthetic needle for medical use in accordance with the preferred embodiments of the present invention will be described.

The epidural needle 1 shown in FIG. 2 is inserted into the patient and advanced onto the epidural space without puncturing the dura mater. It is then confirmed that the bar member 16 of the length adjusting mechanism 10 is in the disengaged position between the engaging projection 17 and the circumferential grooves 45 of the engaging portion 44 of the anesthetic needle 40. The anesthetic needle 40 is inserted from the side of the proximal end portion into the length adjusting mechanism 10 of the epidural needle 1. The anesthetic needle 40 is further inserted into the needle 2. The anesthetic needle 40 is still further advanced until the dura mater is then punctured with the distal end of the needle 41 of the anesthetic needle 40 and the needle 41 reaches the subarachnoid space. When it is felt that the distal end of the needle 41 has been inserted into the subarachnoid space, the bar member 16 shown in FIG. 6 is pushed in to engage the engaging projection 17 with the circumferential grooves 45 of the engaging portion 44 of the anesthetic needle 40.

At this time, it is possible to directly view the engaging portion 44 from the outside through the window portion 14, allowing the position of the engaging portion 44 and the like to be confirmed easily. Further, since the length adjusting mechanism 10 is formed from the transparent material, the position of the engaging portion 44 and the like can be checked even more easily. In addition, the scale 15 for providing a reading of the axial movement of the anesthetic needle 40, if provided near the window portion 14 of the length adjusting mechanism 10, should allow the position of the engaging portion 44 and the like to be determined numerically.

A stylet not shown is then pulled out from the anesthetic needle 40 and it is confirmed if there is a flashback from the proximal end of the anesthetic needle 40. If the flashback is not confirmed, the flashback is confirmed again by turning the anesthetic needle 40 in units of, for example, 90°. If the flashback cannot still be confirmed, the bar member 16 shown in FIG. 6 is pushed in the opposite direction so that the engaging projection 17 is temporarily disengaged from the circumferential grooves 45 of the engaging portion 44 of the anesthetic needle 40. The anesthetic needle 40 is then further inserted and the flashback is confirmed again. When the flashback has been confirmed, the anesthetic is delivered through the anesthetic needle 40. The anesthetic needle 40 is pulled out and an epidural catheter not shown is inserted into the epidural space through the epidural needle 1 as necessary.

As described in the foregoing, in the anesthetic needle for medical use in accordance with the preferred embodiment of the present invention, the axial movement of the anesthetic needle 40 relative to the epidural needle 1 can be inhibited by engaging the engaging projection 17 with the circumferential grooves 45 of the engaging portion 44 after the position of the anesthetic needle 1 has been adjusted. Since the engaging projection 16 can freely slide in the peripheral direction with the circumferential grooves 45, the anesthetic needle 40 can be rotated relative to the epidural needle 1. This rotation of the anesthetic needle 40 is smooth, since there exists substantially a point-to-point contact between the engaging projection 16 and the circumferential grooves 45. Moreover, the engaging portion 44 of the anesthetic needle 40 is loosely fitted within the length adjusting mechanism 10. This results in the sliding pressure between the length adjusting mechanism 10 and the engaging portion 44 being small. This, in turn, helps the practitioner gain very easily a positive sense that the anesthetic needle 40 has punctured into the subarachnoid space.

In addition, there exists only the needle 41 inside the engaging portion 44. This makes it possible to form a thin and lightweight engaging portion 44, thus allowing the anesthetic needle 40 to be smoothly rotated with an exquisite tactile sense. The anesthetic needle for medical use in accordance with the preferred embodiments of the present invention thus allows the practitioner to gain a positive sense that the anesthetic needle 1 has punctured into the subarachnoid space. The anesthetic needle for medical use in accordance with the present invention can also be rotated smoothly with an exquisite tactile sense, while inhibiting the axial movement of the anesthetic needle 40 relative to the epidural needle 1 after the position of the anesthetic needle 40 has been adjusted. Overall, the anesthetic needle for medical use in accordance with the present invention is extremely easy to use.

The anesthetic needle for medical use described in the foregoing may still be configured as detailed in the following.

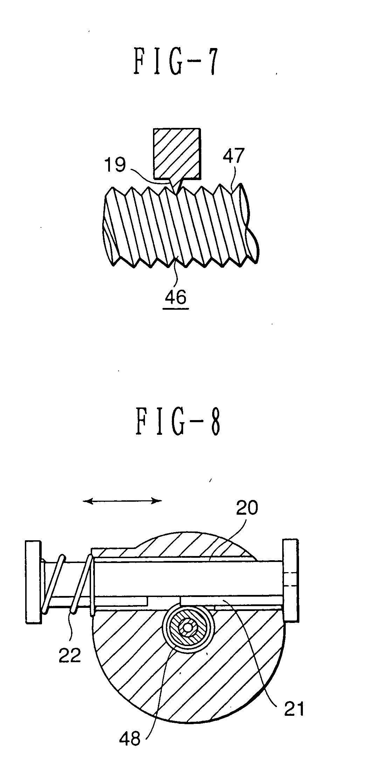

As shown in FIG. 7, circumference grooves 47 of an engaging portion 46 of the anesthestic needle may be formed into external threads. If the circumference grooves 47 are formed into the external threads, an engaging projection 19 is engaged with the circumference grooves 47; as the anesthetic needle is rotated, the engaging projection 19 slides along the circumferential grooves 47, thus moving the axial position of the anesthetic needle slightly relative to the epidural needle. This allows the insertion position of the anesthetic needle to be fine-adjusted.

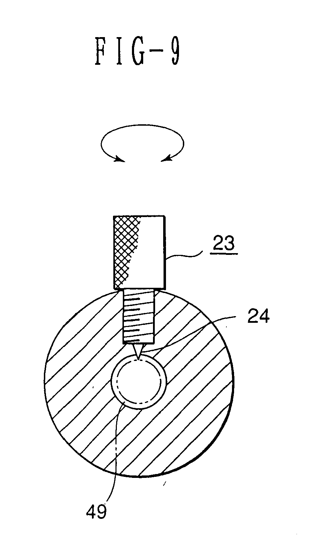

Referring to FIG. 8, a spring 22 may be installed on one end portion of a bar member 20 of the length adjusting mechanism. The spring 22 then urges the bar member 20 toward the engaging position. Through this arrangement, an engaging projection 21 can be disengaged from an engaging portion 48 by pushing the bar member 20 so as to overcome the tension of the spring 22. When the bar member 20 is released, the tension of the spring 22 causes the engaging projection 21 to be automatically engaged with the engaging portion 48. This retains a positive engagement between the engaging projection 21 and the engaging portion 48, making the anesthetic needle for medical use even easier to use.

Referring to FIG. 9, a screw member (a second engaging member) 23 having external threads may be disposed, screwed into the length adjusting mechanism so as to be radially advancing or retracting from the outside of the length adjusting mechanism. An engaging projection 24 is provided in a protruding manner on a distal end portion of the screw member 23. When the screw member 23 is turned, the engaging projection 24 moves between the engaging position and the disengaging position. The engaging projection 24 is thereby engaged with, or disengaged from, an engaging portion 49.

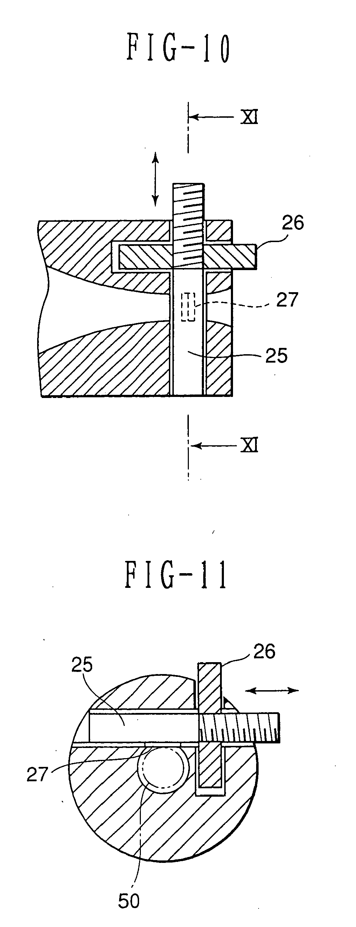

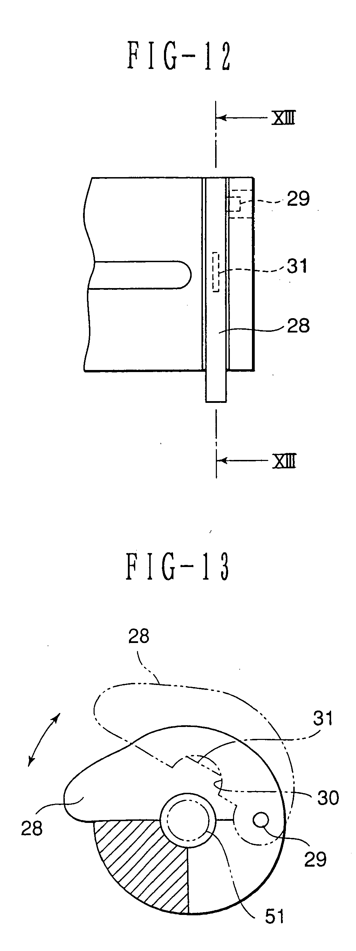

Referring now to FIGS. 10 and 11, the length adjusting mechanism may be provided with the following components. Specifically, one of the components may be a bar member (a third engaging member) 25 that crosses an engaging portion 50 and is inserted in the length adjusting mechanism so as to be slidable, but not rotatable, therein. The other component may be a rotary plate 26 (a third engaging member) that has screw threads therein for engaging the bar member 25 and turns for moving the bar member 25. An engaging projection 27 is provided in a protruding manner on the side of an engaging portion 50 of the bar member 25. As the rotary plate 26 is rotated, the engaging projection 27 moves between the engaging position and the disengaging position. The engaging projection 27 is thereby engaged with, or disengaged from, the engaging portion 50.

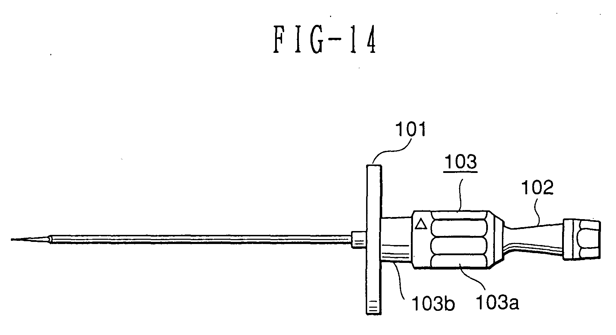

Referring now to FIGS. 12 and 13, a sheet member (a fourth engaging member) 28 that crosses an engaging portion 51 and is vertically oscillatably journaled about a pin shaft 29 may be disposed in the length adjusting mechanism. A concave portion 30 of an inner semi-circular shape is formed on the side of the engaging portion 51 of the sheet member 28. An engaging projection 31 is provided in a protruding manner at an upper portion on the concave portion 30 on the side of the engaging portion 51. When the sheet member 28 is oscillated vertically, the engaging projection 31 moves between the engaging position and the disengaging position. The engaging projection 31 is thereby engaged with, or disengaged from, the engaging portion 51.

### Industrial Applicability

As described in the foregoing, the anesthetic needle for medical use in accordance with the preferred embodiments of the present invention allows the practitioner to gain a positive sense that the anesthetic needle has punctured into the subarachnoid space. The anesthetic needle for medical use in accordance with the present invention can also be rotated smoothly with an exquisite tactile sense, while inhibiting the axial movement of the anesthetic needle relative to the epidural needle after the position of the anesthetic needle has been adjusted. The anesthetic needle for medical use in accordance with the present invention is therefore extremely easy to use and can be widely applied to anesthetic medical care.

## Claims

1. An anesthetic needle for medical use, comprising:
an epidural needle (1), said epidural needle having at a proximal end portion a length adjusting mechanism (10) of a tube shape extending in an axial direction; and
an anesthetic needle (40) rotatably inserted in said epidural needle from a side of the proximal end portion, said anesthetic needle having at a proximal end portion an engaging portion (44, 46, 48, 49, 50, 51) of a circular tube shape loosely fitted within said length adjusting mechanism, said engaging portion having a needle (41) passing at a center portion and a plurality of circumferential grooves (45, 47) axially juxtaposed on an outer peripheral surface, said length adjusting mechanism having therein an engaging projection (17, 19, 21, 24, 27, 31) inhibiting axial movement of said anesthetic needle through engagement with said circumferential grooves, while being circumferentially slidable with said circumferential grooves.

2. The anesthetic needle for medical use according to claim 1, wherein said epidural needle (1) and said length adjusting mechanism (10) are coupled together by fitting a convex portion (4) formed on the proximal end portion of said epidural needle into a concave portion (11) formed on a distal end portion of said length adjusting mechanism.

3. The anesthetic needle for medical use according to claim 1 or 2, wherein said circumferential grooves (45, 47) are formed annularly or into external threads.

4. The anesthetic needle for medical use according to any of claims 1 to 3, wherein said length adjusting mechanism (10) is provided with a first engaging member (16, 20) that crosses said engaging portion (44, 46, 48) and is inserted into said length adjusting mechanism movably between an engaging position and a disengaging position, and said engaging projection (17, 19, 21) is provided in a protruding manner on a side of said engaging portion of said first engaging member.

5. The anesthetic needle for medical use according to claim 4, wherein said first engaging member (20) is urged toward said engaging position by a spring (22).

6. The anesthetic needle for medical use according to any of claims 1 to 3, wherein said length adjusting mechanism is provided with a second engaging member (23) shaped into external threads and screwed into said length adjusting mechanism so as to radially advance or retract between the engaging position and the disengaging position, and said engaging projection (24) is disposed on a distal end portion of said second engaging member.

7. The anesthetic needle for medical use according to any of claims 1 to 3, wherein said length adjusting mechanism is provided with a third engaging member including a bar member (25) that crosses said engaging portion (50) and is inserted into said length adjusting mechanism movably between the engaging position and the disengaging position and a rotary plate (26) that has screw threads thereon for engaging said bar member and turns for moving said bar member, and said engaging projection (27) is disposed on a side of said engaging portion of said bar member.

8. The anesthetic needle for medical use according to any of claims 1 to 3, wherein said length adjusting mechanism is provided with a fourth engaging member (28) that crosses said engaging portion (51) and is journaled in said length adjusting mechanism oscillatably between the engaging position and the disengaging position, and said engaging projection (31) is disposed on a side of said engaging portion of said fourth engaging member.

9. The anesthetic needle for medical use according to any of claims 1 to 8, wherein said length adjusting mechanism (10) is provided with a window portion (14), through which said engaging portion (44) can be viewed from an outside.

10. The anesthetic needle for medical use according to any of claims 1 to 9, wherein said length adjusting mechanism (10) is formed from a transparent material.

11. The anesthetic needle for medical use according to claim 9 or 10, wherein said length adjusting mechanism (10) is provided with a scale for providing a reading of axial movement of said anesthetic needle (40).
